# EUROPEAN PATENT SPECIFICATION

(11) **EP 3 473 221 B1**
(45) Date of publication and mention of the grant of the patent: **01.09.2021**
(21) Application number: 17814793.0
(22) Date of filing: 20.06.2017
(51) Int. Cl.: A61F 13/22, A61F 13/51, A61F 13/20

(54) **TAMPON**
TAMPON
TAMPON

(30) Priority: 20.06.2016 ES 201600438 U
(43) Date of publication of application: 24.04.2019
(73) Proprietor: Vendrell Vila, Ramón, 08660 Balsareny Barcelona (ES)
(72) Inventor: FONT CASELLES, Ramon, 08241 Manresa / Barcelona (ES)
(74) Representative: Gallego Jiménez, José Fernando
(86) International application number: PCT/ES2017/000074
(87) International publication number: WO 2017/220824

(56) References cited:
- EP-A1- 2 749 261
- US-A1- 2005 096 620
- US-A1- 2012 101 462

## Description

### OBJECT OF THE INVENTION

The object of the invention is a tampon, particularly for feminine hygiene; which comprises a substantially cylindrical absorbent body that is peripherally compressed and contained in a wrapper or in an applicator device, with an absorbent body formed by a rolled rectangular strip of absorbent material, and said absorbent body comprises: a front end in the direction of insertion of the tampon, a rear end from which extends a removal string and a lateral or peripheral surface.

### FIELD OF APPLICATION OF THE INVENTION

This invention is applicable in the field dedicated to the manufacture of protective products for intimate feminine hygiene.

### STATE OF THE ART

There are currently in the market several tampons for feminine hygiene that are generally comprised of an absorbent body with a cylindrical configuration which is compressed inside an applicator device or in a wrapper, said absorbent body being formed by a rolled strip of absorbent material that comprises an absorbent core, which includes absorbent fibres.

These tampons generally have the drawback that the absorbent fibres in the core are arranged on the exterior of the absorbent body of the tampon.

This external arrangement of the absorbent fibres means that when the tampon is being used, the fibres are unprotected and remain in contact with the vagina of the user, causing irritation, particularly in the final days of menstruation, since said absorbent fibres adhere to the user due to the lower amount of discharge, drying out and making it difficult to remove the tampon.

In the tampons currently in the market the absorbent fibres are exposed in the entirety of the lateral surface of the cylindrical body and/or in the front and rear ends of the tampon, causing the aforementioned problems.

Patent document EP2749261A1 discloses a tampon made of an absorbent composite material comprising an absorbent material formed of a liquid-absorptive sponge wrapped with a liquid-permeable wrapping material held together in a rolled position through a sheet strip used for sealing.

The applicant of the invention is unaware of the existence of tampons with the suitable characteristics required to address these drawbacks.

### DESCRIPTION OF THE INVENTION

The tampon that is the object of the invention, applicable in feminine hygiene, is of the kind that comprises a substantially cylindrical absorbent body that is peripherally compressed and contained in a wrapper or in an applicator device, with an absorbent body formed by a rolled rectangular strip of absorbent material; said absorbent body comprises a front end in the direction of insertion of the tampon, a rear end from which extends a removal string and a lateral surface.

This tampon has a series of characteristics designed to address the technical problem mentioned above; and more specifically to prevent the absorbent fibres from coming into contact with the body of the user and therefore adhering to the body, causing irritation or making it difficult to remove the tampon.

In order to achieve this and according to the invention, in the solution provided the entirety of the outer surface of the absorbent body corresponding to the front end, the rear end and the lateral surface thereof, are formed of a first layer of non-woven absorbent fabric.

In this first layer of non-woven fabric, the fibres comprising the fabric are joined together by means of punching, for example, preventing them from coming loose and adhering to the body of the user, causing irritation or making it difficult to remove the tampon.

In a first embodiment of this invention the strip of absorbent material is formed by the aforementioned first layer of non-woven absorbent fabric. In this embodiment the absorbent body lacks an absorbent core, which is intended for those cases in which tampons are manufactured with a low absorption capacity.

In this embodiment the first layer of non-woven fabric comprises: a central longitudinal portion of a width equal to the length of the tampon, with an outer side and an inner side in relation to the direction in which it is rolled up; and two lateral longitudinal wings folded over the inner side of the central longitudinal portion.

In a second embodiment of the invention, the strip of absorbent material also comprises an absorbent core that can have a variable thickness depending on the desired absorption capacity of the tampon.

This absorbent core, which is formed by absorbent fibres such as cotton, viscose or similar, is located between the central portion and the folded wings of the first layer of non-woven fabric; with the outer side and longitudinal edges thereof being completely covered by said first layer of non-woven fabric; and at least two lateral strips of the inner side thereof being covered by the wings of the aforementioned first layer of non-woven fabric.

In this way, when the absorbent strip of material is rolled up in order to form the body of the tampon, the absorbent core formed by the absorbent fibres is completely covered by the first layer of non-woven fabric, with said first layer preventing the absorbent fibres of the absorbent core from adhering to the user when it is being used, and causing her irritation.

According to other characteristics of the invention, independently of whether the absorbent body comprises an absorbent core, the absorbent body is held in the rolled position by means of pressurised water jets applied on the strip of absorbent material itself after being rolled up.

### DESCRIPTION OF THE FIGURES

To complement the description being made, and to make it easier to understand the characteristics of the invention, this descriptive report is accompanied by a set of drawings in which, for illustrative purposes only, and without limitation, the following has been represented:
Figure 1 shows a schematic perspective view of the tampon that is the object of the invention contained in an applicator device.
Figure 2 shows a perspective view of a first example embodiment of the substantially cylindrical absorbent body of the tampon, formed by a rolled rectangular strip of absorbent material and comprising a first layer of non-woven absorbent fabric.
Figure 3 shows a perspective view of the development of the absorbent strip comprising the body of the tampon in Figure 2, which is provided on one of the ends thereof with a supporting strip, which is secured by means of heat.
Figure 4 shows a perspective view of a variation of the embodiment of the absorbent body intended to be rolled up in order to form the body of the tampon, comprising in this case an absorbent core of absorbent fibres and also provided on one of the ends thereof with a supporting strip.

### PREFERRED EMBODIMENT OF THE INVENTION.

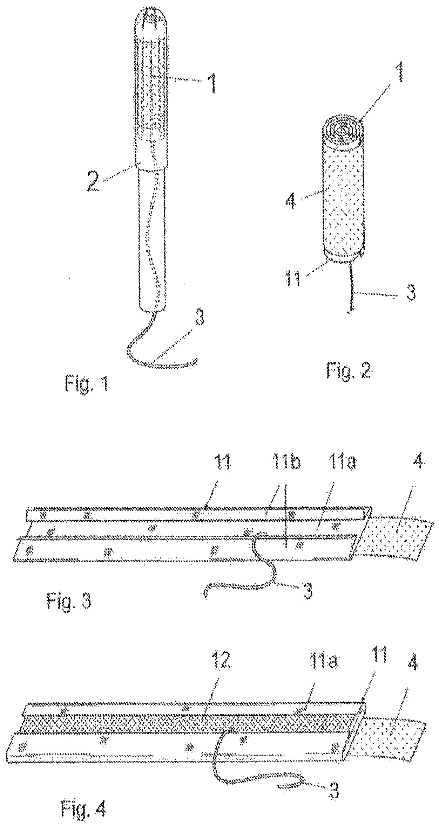

The example shown in Figure 1 shows the tampon that is the invention, which comprises a substantially cylindrical absorbent body (1) intended to be peripherally compressed, and in this particular case to be contained in an applicator device (2).

The absorbent body (1) is formed by a rolled rectangular strip of direction of insertion of the tampon, a rear end (1b) from which extends a removal string (3), and a lateral surface (1e).

The strip of absorbent material intended to be rolled up in order to form the absorbent body (1) comprises a first layer (11) of absorbent non-woven fabric comprising: a central longitudinal portion (11a) of a width equal to the length of the absorbent body (1) of the tampon, with an outer side and an inner side in relation to the direction in which it is rolled up; and two lateral longitudinal wings (11b) folded over the inner side of the central portion (11a), wherein the fibres comprising said first layer (11) of absorbent non-woven fabric are joined together by means of punching, preventing them from coming loose and adhering to the body of the user; and said absorbent body (1) is held in a rolled position by means of pressurised water jets applied on the strip of absorbent material itself, after it has been rolled up.

In this way, in the rolled position shown in Figure 2, the entirety of the outer surface of the absorbent body (1) is formed by the aforementioned first layer (11) of non-woven fabric.

In the example shown in Figures 2 and 3, an alternative embodiment further includes on one of the ends of the absorbent material a supporting strip (4), which is made of polypropylene in this case, which is secured by means of heat on the outer surface of the absorbent body (1), after it has been rolled up, preventing it from accidentally unrolling and also limiting the lateral expansion capacity thereof when the tampon is being used.

In this embodiment the absorbent body (1) is only comprised of a first layer (11) of non-woven fabric that can have a variable thickness depending on the desired absorption capacity.

In the variation of the embodiment shown in Figure 4, the strip of absorbent material intended to form the absorbent body (1) comprises, in addition to the aforementioned first layer (11) of non-woven fabric, an absorbent core (12) that can be thicker or thinner depending on the absorption capacity of the desired tampon.

This absorbent core (12), which is formed by absorbent fibres of cotton, viscose or similar; is arranged between the central portion (11a) and the folded wings (11b) of the first layer (11) of non-woven fabric. This arrangement of the absorbent core (12) causes the outer side and longitudinal edges thereof to be entirely covered by the first layer of non-woven fabric; and also causes at least two lateral strips of the inner side thereof to be covered by the wings (11b) of the first layer (11) of non-woven fabric.

In this way, when the first layer (11) is rolled up with the absorbent core (12) in order to form a cylindrical absorbent body (1), the entirety of the outer surface of said absorbent body (1) is formed by the first layer (11) of non-woven fabric, completely hiding the fibres of the absorbent core (12), which prevents said absorbent fibres from coming into direct contact with the user, solving the problems described above.

Having described sufficiently the nature of the invention, and the examples of a preferred embodiment, it can be stated for the relevant effects that the materials, shape, size and arrangement of the elements described may be modified, provided this does not mean an alteration in the essential characteristics of the invention for which the claims are set out below.

## Claims

1. Tampon, particularly for feminine hygiene, comprising a substantially cylindrical absorbent body (1) that is peripherally compressed and contained in a wrapper or in an applicator device (2), said absorbent body (1having a front end (1a) in the direction of insertion of the tampon; a rear end (1b) from which extends a removal string (3), and a lateral surface (1e);
**characterised in that**
said absorbent body (1) is formed by a rolled rectangular strip of absorbent material comprising a first layer (11) of non-woven absorbent fabric wherein the fibres comprising said first layer (11) of non-woven absorbent fabric are joined together by means of punching, preventing them from coming loose and adhering to the body of the user; and
said absorbent body (1) is held in a rolled position by means of pressurised water jets applied on the strip of absorbent material itself, after it has been rolled up.

2. Tampon according to claim 1, **characterised in that** the strip of absorbent material comprises a first layer (11) of non-woven absorbent fabric that comprises: a central longitudinal portion (11a) with a width equal to the length of the absorbent body (1) of the tampon, with an outer side and an inner side in relation to the direction in which it is rolled up; and two lateral longitudinal wings (11b) folded over the inner side of the central portion (11a).

3. Tampon according to claim 2, **characterised in that** the strip of absorbent material comprises an absorbent core (12) of a variable thickness depending on the desired absorption capacity of the tampon that is formed by absorbent fibres; said absorbent core (12) is arranged between the central portion (11a) and the folded wings (11b) of the first layer (11) of the non-woven absorbent fabric; with the outer side and longitudinal edges thereof being completely covered by said first layer (11) of non-woven absorbent fabric; and at least two lateral strips of the inner side thereof being covered by the wings (11b) of the first layer of non-woven absorbent fabric

## Patentansprüche

1. Tampon, insbesondere für die Intimpflege, umfassend einen im Wesentlichen zylindrischen absorbierenden Körper (1), der umlaufend komprimiert und in einer Umhüllung oder in einer Applikatorvorrichtung (2) enthalten ist, wobei der absorbierende Körper (1 ein vorderes Ende (1a) in Einführrichtung des Tampons aufweist; ein hinteres Ende (1b), von dem sich ein Rückholfaden (3) und eine laterale Oberfläche (1e) erstrecken;
**dadurch gekennzeichnet, dass**
der absorbierende Körper (1) durch einen aufgerollten rechteckigen Streifen aus absorbierendem Material gebildet wird, der eine erste Schicht (11) aus absorbierendem Vliesstoff umfasst, wobei die Fasern, welche die erste Schicht (11) aus absorbierendem Vliesstoff umfassen, durch Stanzmittel miteinander verbunden sind, um zu verhindern, dass diese sich ablösen und am Körper des Benutzers haften; und
dadurch, dass der absorbierende Körper (1) in einer aufgerollten Position mittels druckbeaufschlagter Wasserstrahlen gehalten wird, die auf den Streifen des absorbierenden Materials selbst aufgebracht werden, nachdem dieser aufgerollt wurde.

2. Tampon nach Anspruch 1, **dadurch gekennzeichnet, dass** der Streifen aus absorbierendem Material eine erste Schicht (11) aus absorbierendem Vliesstoff umfasst, die umfasst: einen zentralen längsverlaufenden Abschnitt (11a) mit einer Breite gleich der Länge des absorbierenden Körpers (1) des Tampons, mit einer Außenseite und einer Innenseite in Bezug auf die Aufrollrichtung; und zwei laterale längsverlaufende Flügel (11b), die über die Innenseite des zentralen Abschnitts (11a) gefaltet sind.

3. Tampon nach Anspruch 2, **dadurch gekennzeichnet, dass** der Streifen aus absorbierendem Material einen absorbierenden Kern (12) von variabler Dicke je nach der gewünschten Absorptionskapazität des Tampons umfasst, der durch die absorbierenden Fasern gebildet wird; der absorbierende Kern (12) zwischen dem zentralen Abschnitt (11a) und den gefalteten Flügeln (11b) der ersten Schicht (11) des absorbierenden Vliesstoffs angeordnet ist; wobei die Außenseite und die längsverlaufenden Ränder vollständig durch die erste Schicht (11) des absorbierenden Vliesstoffs bedeckt sind; und dadurch, dass mindestens zwei laterale Streifen der Innenseite davon durch die Flügel (11b) der ersten Schicht aus absorbierendem Vliesstoff bedeckt sind.

## Revendications

1. Tampon, notamment pour l'hygiène féminine, comprenant un corps absorbant sensiblement cylindrique (1) qui est comprimé périphériquement et contenu dans une enveloppe ou dans un dispositif applicateur (2), ledit corps absorbant (1ayant une extrémité avant (1a) dans le sens d'insertion du tampon ; une extrémité arrière (1b) à partir de laquelle s'étend un cordon de retrait (3), et une surface latérale (1e) ;
**caractérisé en ce que**
ledit corps absorbant (1) est formé d'une bande rectangulaire enroulée de matériau absorbant comprenant une première couche (11) de tissu absorbant non tissé dans lequel les fibres comprenant ladite première couche (11) de tissu absorbant non tissé sont jointes ensemble par des moyens de poinçonnage, les empêchant de se détacher et d'adhérer au corps de l'utilisatrice ; et
ledit corps absorbant (1) est maintenu en position enroulée au moyen de jets d'eau sous pression appliqués sur la propre bande de matériau absorbant, après avoir été enroulée.

2. Tampon selon la revendication 1, **caractérisé en ce que** la bande de matériau absorbant comprend une première couche (11) de tissu absorbant non tissé qui comprend : une partie longitudinale centrale (11a) d'une largeur égale à la longueur du corps absorbant (1) du tampon, avec un côté externe et un côté interne par rapport au sens dans lequel elle est enroulée ; et deux ailes longitudinales latérales (11b) repliées sur le côté interne de la partie centrale (11a).

3. Tampon selon la revendication 2, **caractérisé en ce que** la bande de matériau absorbant comprend une âme absorbante (12) d'une épaisseur variable en fonction de la capacité d'absorption souhaitée du tampon qui est formée de fibres absorbantes; ladite âme absorbante (12) est agencée entre la partie centrale (11a) et les ailes repliées (11b) de la première couche (11) du tissu absorbant non tissé ; avec le côté externe et les bords longitudinaux de celle-ci étant complètement recouverts par ladite première couche (11) de tissu absorbant non tissé ; et au moins deux bandes latérales du côté interne de celle-ci étant recouvertes par les ailes (11b) de la première couche de tissu absorbant non tissé.
